Europäisches Patentamt

European Patent Office

Office européen des brevets

(11) Veröffentlichungsnummer: **0 522 067 B1**

(12) **EUROPÄISCHE PATENTSCHRIFT**

(19)

(45) Veröffentlichungstag der Patentschrift: **28.06.95**

(21) Anmeldenummer: **91907710.7**

(22) Anmeldetag: **20.03.91**

(86) Internationale Anmeldenummer:
**PCT/EP91/00543**

(87) Internationale Veröffentlichungsnummer:
**WO 91/14665 (03.10.91 91/23)**

(51) Int. Cl.⁶: **C07C  6/04**, C07C 6/06,
B01J 31/12

(54) **VERWENDUNG VON ORGANISCHEN DERIVATEN VON RHENIUMOXIDEN ALS KATALYSATOREN ZUR ÄTHENOLYTISCHEN METATHESE OLEFINISCHER VERBINDUNGEN UND VERFAHREN ZUR ÄTHENOLYTISCHEN METATHESE VON OLEFINISCHEN VERBINDUNGEN MIT HILFE DIESER KATALYSATOREN.**

(30) Priorität: **28.03.90 DE 4009910**

(43) Veröffentlichungstag der Anmeldung:
**13.01.93 Patentblatt  93/02**

(45) Bekanntmachung des Hinweises auf die
Patenterteilung:
**28.06.95 Patentblatt  95/26**

(84) Benannte Vertragsstaaten:
**DE FR GB IT**

(56) Entgegenhaltungen:
**EP-A- 0 373 488**
**FR-A- 2 499 083**
**US-A- 3 776 973**
**US-A- 4 180 524**

(73) Patentinhaber: **HOECHST AKTIENGESELL-
SCHAFT**

**D-65926 Frankfurt (DE)**

(72) Erfinder: **HERRMANN, Wolfgang, Anton
Gartenstrasse 69
D-8050 Freising (DE)**
Erfinder: **WAGNER, Werner
Schlierseestrasse 29
D-8000 München (DE)**

EP 0 522 067 B1

## Beschreibung

Die vorliegende Erfindung bezieht sich auf ein Verfahren zur sogenannten äthenolytischen Metathese (Äthenolyse) von nicht-funktionalisierten und funktionalisierten Olefinen unter Verwendung von organischen Derivaten von Rheniumoxiden, sowie auf die Verwendung von solchen Derivaten als Katalysatoren zu dieser Äthenolyse.

Der Begriff Äthenolyse bezeichnet die Spaltung von olefinischen Verbindungen in Gegenwart des Olefin-Grundkörpers Äthylen, und zwar in der Weise, daß die Doppelbindungen des betreffenden Olefins und des Äthylens auseinandergebrochen werden und sich die resultierenden Bruchstücke in statistischer Weise zu neuen olefinischen Verbindungen vereinigen. Dabei entsteht ein einziges Produkt dann, wenn man ein symmetrisches Monoolefin einsetzt. Aus einem unsymmetrischen Monoolefin erhält man zwei verschiedene Produkte. Bei Einsatz von Di- und Oligoolefinen erhöht sich die Zahl der Produkte entsprechend. Zur Äthenolyse von Olefinen gehört im weiteren Sinne auch die Ringöffnung von Cycloolefinen, wobei man aus einem Cycloolefin mit n Ringgliedern durch Äthenolyse ein $\omega,\omega'$-Diolefin mit n + 2 Kettengliedern erhält.

Äthenolytische Olefin-Spaltungen sind für die Herstellung von Fein- und Großchemikalien seit geraumer Zeit von industriellem Interesse. So sind das Phillips-Verfahren zur Herstellung von Neohexen (3,3-Dimethyl-1-buten) und das Shell-Verfahren zur Herstellung von $\omega,\omega'$-Diolefinen, die als Vernetzer bei der Olefin-Polymerisation oder zur Herstellung bifunktioneller Verbindungen technisch wichtig sind, industrielle Beispiele der katalytischen Olefin-Äthenolyse.

Während man bei der herkömmlichen Olefin-Metathese, zumeist "Selbstmetathese" genannt, den Zweck verfolgt, ein unsymmetrisches Olefin in zwei andere Olefine mit kürzerer oder längerer C-Atomkette überzuführen oder ein cyclisches Olefin ringöffnend zu di- bzw. polymerisieren, unterscheidet sich die Äthenolyse davon dergestalt, daß man grundsätzlich endständige Olefine ($\alpha$-Olefine) erzeugt, wobei die Fragmente, die durch den Bruch des Ausgangsolefins an der Doppelbindung entstehen, jeweils um eine $CH_2$-Gruppe verlängert werden. Die Äthenolyse innenständiger Olefine ist somit das Gegenstück zur Selbstmetathese von $\alpha$-Olefinen.

Die Äthenolyse ist praktisch immer eine Druckreaktion und insofern auch verfahrenstechnisch von der herkömmlichen Olefin-Metathese unterschieden. In aller Regel hat man auch unterschiedliche Katalysatoren für die beiden Prozesse verwendet.

Es wurde nun überraschend gefunden, daß sich Verbindungen der allgemeinen Formel $R^1{}_a Re_b O_c$ (I), worin a 1 bis 6, b 1 bis 4 und c 1 bis 14 sind und die Summe von a, b und c so ist, daß sie der 5- bis 7-Wertigkeit des Rheniums gerecht wird mit der Maßgabe, daß c nicht größer als 3•b ist, und worin $R^1$ einen Alkylrest mit 1 bis 9 C-Atomen, einen Cycloalkylrest mit 5 bis 10 C-Atomen oder einen Aralkylrest mit 7 bis 9 C-Atomen darstellt, wobei $R^1$ wenigstens teilweise fluoriert sein kann, die Verbindungen nicht mehr als drei Gruppen mit mehr als 6 C-Atomen je Rheniumatom enthalten und an das C-Atom in $\alpha$-Stellung noch wenigstens ein Wasserstoffatom gebunden ist, die auf oxydische Trägermaterialien aufgebracht sind, als Katalysatoren (Heterogenkatalysatoren) für die Äthenolyse (äthenolytische Metathese) kettenförmiger und zyklischer, nichtfunktionalisierter und funktionalisierter Olefine eignen. Bei der Verwendung dieser Katalysatoren kann sogar auf die aus vielen Gründen nachteilige Verwendung zusätzlicher Aktivatoren ("Cokatalysatoren") verzichtet werden, wie dies nach älteren Verfahren erforderlich ist.

Gegenstand der Erfindung ist auch ein Verfahren zur Äthenolyse von olefinischen Verbindungen, das dadurch gekennzeichnet ist, daß man nicht-funktionalisierte oder funktionalisierte Olefine des Typs $YCZ = CZ-(CX_2)_n R^2$ (II), worin n eine ganze Zahl von 1 bis 28, X H oder F ist, Y H oder Alkyl mit 1 bis 10 C-Atomen ist und Z H oder einen nichtaromatischen Kohlenwasserstoffrest mit 1 bis 6 C-Atomen darstellt, Y und Z jedoch nicht gleichzeitig Wasserstoff sind, und der Substituent $R^2$ H, Alkyl, Halogen, $COOR^3$ oder $OR^4$ ist, worin $R^3$ und $R^4$ Alkyl mit 1 bis 15, vorzugsweise 1 bis 6, Kohlenstoffatomen oder Phenyl sind, das am Ring noch 1 bis 3 Substituenten enthalten kann oder worin $R^4$ Trialkyl-silyl $R^5_3$ Si ist, worin $R^5$ Alkyl mit 1 bis 5, vorzugsweise 1 bis 3, C-Atomen darstellt, mit Äthylen an Katalysatoren umsetzt, die aus oxydischen Trägermaterialien bestehen, auf die Verbindungen der vorgenannten Art aufgebracht sind.

Wenn Z von H verschieden ist, ist es vorzugsweise offenkettiges Alkyl mit 1 bis 4 C-Atomen. Z als Alkyl ist z. B. Cyclohexyl, vorzugsweise aber offenkettiges Alkyl mit 1 bis 4 C-Atomen. Wenn Z Phenyl ist, können die Substituenten Halogen, z. B. Fluor, Chlor oder Brom, $NO_2$, $NR^6 R^7$, $OR^8$ und/oder Alkyl sein. Die Reste $R^6$, $R^7$ und $R^8$ sind gleich oder verschieden und können Wasserstoff oder Alkyl mit 1 bis 4 C-Atomen sein. $R^2$ als Halogen kann Fluor, Chlor, Brom oder Jod sein. Die beiden Z können gleich oder verschieden sein. Wenn Z Wasserstoff und $R^2$ Halogen ist, ist dieses bevorzugt Brom. Verbindungen, in denen wenigstens ein X = F ist, sind z. B.

1,6-Di-(perfluor-n-hexyl)-hexen(3) der Formel

$(n-C_6F_{13})-CH_2-CH_2-CH=CH-CH_2-CH_2-(n-C_6F_{13})$

und Perfluorpropen $C_3F_6$. Die Gliederzahl n variiert darin vorzugsweise im Bereich von 1 bis 12 und insbesondere bis 8.

Die Katalysatoren sind also nicht nur bei Olefinen mit Z = H, sondern auch bei der Äthenolyse teilweise fluorierter Olefine wirksam. Sie eignen sich auch für die Äthenolyse innenständiger, funktionalisierter Olefine der Formel $R^9CH=CH-(CH_2)_nR^{10}$ (III), worin $R^9$ ein verzweigte oder zweckmäßig unverzweigter Alkylrest mit 1 bis 12 C-Atomen, $R^{10}$ ein Carboxyalkylrest ist, worin der Alkylrest zweckmäßig 1 bis 4 C-Atome hat und n eine ganze Zahl von 1 bis 10 darstellt. Beispielhaft sei Methyloleat genannt ($R^9$ = n-Octyl, $R^{10}$ = $CO_2CH_3$, n = 7).

Die erfindungsgemäß verwendeten Katalysatoren katalysieren jedoch nicht nur die Äthenolyse offenkettiger Verbindungen, sondern auch die ringöffnende Äthenolyse von Cycloolefinen und cyclischen Kohlenwasserstoffen mit mehreren olefinischen Strukturelementen. Beispiele sind Cycloocten, 1,5-Cyclooctadien und Cycloolefine mit bis zu 20 Kettengliedern, außerdem cyclische Di- und Oligoolefine, die noch heteroatomhaltige Funktionen tragen.

In der Formel I bedeutet $R^1$ eine organische Gruppe, die über ein Kohlenstoffatom, an das noch wenigstens ein Wasserstoffatom gebunden ist, an das Metall Rhenium gebunden ist, und zwar Alkylreste mit 1 bis 9 C-Atomen, Cycloalkyl mit 5 bis 10 C-Atomen, wie Cyclopentyl, Cyclohexyl oder 1-Norbornyl, oder Aralkyl mit 7 bis 9 C-Atomen, wie Benzyl, vorzugsweise aber Methyl. Die Begriffe Alkyl und Cycloalkyl beinhalten naturgemäß, daß diese Gruppen keine Mehrfachbindungen enthalten. In den Verbindungen kommt aus sterischen Gründen die Anwesenheit von mehr als drei Gruppen mit mehr als 6 C-Atomen je Rheniumatom nicht in Betracht; zweckmäßig enthalten die Verbindungen nur höchstens eine solche Gruppe. Der Begriff der Metathese schließt hierbei die Ringöffnung von Cycloolefinen ein.

Von den Verbindungen der Formel I sind das $CH_3ReO_3$, das $(CH_3)_6Re_2O_3$, das $(CH_3)_4 Re_2O_4$ und das Pentamethylcylopentadienylrheniumtrioxyd zwar bekannt, jedoch war die katalytische Wirksamkeit dieser Verbindungen in der Äthenolyse, also in Druckreaktionen des Äthylens, nicht bekannt und ebensowenig zu erwarten wie die der ganzen Verbindungsklasse Die katalytische Wirkung überrascht um so mehr, als das analog aufgebaute (Trimethylstannoxy)rheniumtrioxid $[(CH_3)_3SnO]ReO_3$ katalytisch ebenso unwirksam ist wie alle anderen oxidischen Rheniumverbindungen, so das Dirheniumheptoxid $Re_2O_7$, verschiedene Perrhenate mit dem Anion $[ReO_4]^-$, das Rheniumtrioxid $ReO_3$ und die übrigen Rheniumoxide $Re_2O_5$ und $ReO_2$. Auch $(CH_3)_3SiOReO_3$ ist unwirksam. In den Verbindungen der Formeln I und II bedeutet Alkyl z.B. Methyl, Äthyl, Propyl, Isopropyl, die verschiedenen Butylreste wie n-, sek-, tert.- und Isobutyl, die verschiedenen Pentyl-, Hexyl- und Octylreste, wie den 2-Äthyl-hexylrest.

Die gemäß der vorliegenden Erfindung verwendeten Katalysatoren der Formel I lassen sich auf sehr einfache Weise aus $Re_2O_7$ mit Hilfe üblicher Alkylierungsmittel herstellen. Solche Alkylierungsmittel sind z.B. Bor-, Aluminium-, Cadmium-, Quecksilber- und insbesondere Zink- und Zinnverbindungen. Z. B. setzt man Dirheniumheptoxid in einem wasserfreien, gegenüber den Rheniumverbindungen inerten Lösungsmittel (z.B. Tetrahydrofuran) bei einer Temperatur von -80 bis +60°C, vorzugsweise -30 bis +40°C, mit einer Lösung von $R^1_2$ Zn oder von anderen Alkylierungsmitteln um, wobei $R^1$ die oben angegebene Bedeutung hat, filtriert die so entstehende Suspension über eine Tauchfritte und entfernt vom Filtrat die flüchtigen Anteile. Wenn man besonders aktive Zinkalkyle umsetzt, empfiehlt es sich, bei den niedrigeren Temperaturen innerhalb der angegebenen Bereiche zu arbeiten, um eine selektive Umsetzung zu den gewünschten Rheniumverbindungen zu erhalten und so die Bildung von unerwünschten Nebenprodukten zu reduzieren bzw. zu verhindern. In anderen Fällen arbeitet man zweckmäßig bei einer Temperatur von 0 bis 60°C, vorzugsweise 10 bis 40°C. Die Verbindungen der Formel I können Feststoffe oder Flüssigkeiten sein. Sie sind als solche zwar unempfindlich gegenüber Luft und Feuchtigkeit, jedoch sind sie vor ihrer Verwendung gut zu trocknen. Dies gilt vor allem auch für die Trägermaterialien.

Besonders in Betracht kommen Katalysatoren, die als Rheniumverbindung das leicht zugängliche, bei Raumtemperatur feste Methylrheniumtrioxid $CH_3ReO_3$ enthalten. Als Trägermaterialien eignen sich insbesondere Aluminiumoxid und Kombinationen davon mit Siliziumdioxid (z.B. $SiO_2/Al_2O_3$ im Gewichtsverhältnis 87:13), jedoch auch andere Oxide wie Titan-, Zirkon-, Niob-, Tantal- und Chromoxide für sich oder in Kombination mit Aluminium- und/oder Siliziumdioxid. Das Aluminiumoxid kann je nach Vorbehandlung sauer, neutral oder basisch sein. Die Aktivität dieser Katalysatoren kann erheblich gesteigert werden, wenn die Rheniumverbindungen auf einen durch Erhitzen möglichst weit von Wasser befreiten Träger wie Kiesel-/Aluminiumoxid aufgebracht werden. Enthält nämlich das Trägermaterial größere Feuchtigkeitsmengen, so verringert sich die Aktivität, weil dann die an Rhenium gebundene Alkylgruppe durch das Wasser teilweise als Alkan abgespalten wird, z.B. nach der Gleichung $CH_3ReO_3 + H_2O \rightarrow CH_4 + HReO_4$.

3

Für das erfindungsgemäße Verfahren kann man auf zeit- und energieaufwendige Arbeitsschritte verzichten und dennoch gut reproduzierbare Katalysatoren erhalten.

Die katalytisch wirkende Rheniumverbindung wird für das vorliegende Verfahren vorteilhaft bei Raumtemperatur aus einem Lösungsmittel, vorzugsweise einer Dichlormethan-Lösung, auf den Katalysatorträger, vorteilhaft Kieselgel/Aluminiumoxid, aufgebracht, wobei lediglich der Katalysatorträger vor seiner Verwendung 2 h bei 550 bis 800°C im Stickstoffstrom von Feuchtigkeit befreit wird, damit das Katalysatorsystem nachher seine volle Aktivität entfaltet.

Bei der Äthenolyse mit den erfindungsgemäß verwendeten Katalysatoren ist darauf zu achten, daß Luft und Feuchtigkeit ausgeschlossen werden. Auch die eingesetzten Olefine sind vor ihrer Verwendung zweckmäßig gut zu trocknen, um die Bildung von Alkanen als Nebenprodukten zu vermeiden. Die Äthenolyse wird im allgemeinen bei 3 bis 30, vorzugsweise 5 bis 20 bar Äthylendruck und einer Temperatur von -25 bis +70°C, zweckmäßig +20 bis 65°C durchgeführt.

Die Möglichkeit, bei solch relativ milden Reaktionsbedingungen zu arbeiten, ist ein besonderer Vorteil des erfindungsgemäßen Verfahrens. Es ist aber auch möglich, noch höhere Temperaturen, z. B. bis 100°C anzuwenden oder bei geringerem, z. B. Atmosphärendruck, zu arbeiten. Jedoch sind hiermit gewöhnlich keine Vorteile verbunden.

**Beispiele 1 bis 13 - Äthenolyse mit $(CH_3)ReO_3$**

In einen 250 ml-Laborautoklaven aus Sicherheitsglas (BÜCHI-Glasreaktor) wurde unter Rühren bei der in Tabelle 1 angegebenen Temperatur eine Lösung von 13 mg (0,052 mmol) Methylrheniumtrioxid $CH_3ReO_3$ in 0,5 ml Dichlormethan in eine Suspension von 2000 mg Katalysatorträger $SiO_2/Al_2O_3$ - (Gewichtsverhältnis 87 : 13, Korngröße unter 15 μm; 2 h auf 550°C gehalten) in 50 ml Dichlormethan (über Calciumhydrid getrocknet und unter Stickstoffatmosphäre aufbewahrt) eingetragen. Nach 5-minütigem Rühren wurden 2,5 mmol Olefin eingespritzt (t = 0) und ein aus Tabelle 1 ersichtlicher Äthylendruck aufgepreßt. Nach einer Reaktionszeit von 5 h wurden in der Flüssigphase die in der Tabelle 1 angegebenen Produkte gaschromatographisch nachgewiesen.

**Beispiele 14 bis 17 - Äthenolyse mit $(CH_3)_4 Re_2 O_4$**

Bei der Verwendung dieser Verbindung als Katalysator für die Olefin-Metathese wurde ebenso verfahren wie es in den Beispielen 1 bis 13 für Methylrheniumtrioxid $CH_3ReO_3$ beschrieben ist, nur daß man statt der Lösung von 13 mg Methylrheniumtrioxid nun eine Lösung von 25,4 mg (0,052 mmol) Tetramethyltetraoxodirhenium $(CH_3)_4 Re_2 O_4$ in 0,5 ml Dichlormethan in eine Suspension von 2000 mg des KatalysatorträgerS eintrug. Nach einer Reaktionszeit von 5 h wurden in der Flüssigphase die in Tab. 2 angegebenen Produkte gaschromatographisch nachgewiesen.

4

Tabelle 1

| Äthenolyse nicht-funktionalisierter und funktionalisierter offenkettiger und cyclischer Olefine mit $(CH_3)ReO_3$ | | | |
|---|---|---|---|
| Beispiel/Ausgangsmaterial | Reaktionsbedingungen | Produkte | Ausbeuten [a] (%) |
| 1) cis/trans-3-Hepten | 10 bar/40°C | 1-Buten<br>1-Penten | 39<br>39 |
| 2) β-Di-iso-buten* | 15 bar/40°C | 3,3-Dimethyl-1-buten<br>Isobuten | 46<br>45 |
| 3) 1,3-Cyclohexadien | 8 bar/28°C | 1,5-Hexadien<br>1,3-Butadien<br>1,4,7-Octatrien | 42<br>40<br>7 |
| 4) Cyclododecen | 8 bar/30°C | 1,13-Tetradecadien | 98 |
| 5) Cycloocten | 8 bar/30°C | 1,9-Decadien | 91 |
| 6) 1,5-Cyclooctadien | 15 bar/35°C | 1,5-Hexadien<br>1,5,9-Decatrien | 72<br>12 |
| 7) Cyclopenten | 10 bar/28°C | 1,6-Heptadien | 80 |
| 8) 1,9-Cyclohexadecadien | 15 bar/40°C | 1,9,17-Octadecatrien<br>1,9-Decadien | 12<br>66 |
| 9) α,α'-Diphenylstilben | 18 bar/65°C | 1,1-Diphenyläthylen | 53 |
| 10) 2-Norbornen | 13 bar/30°C | 1,4-Divinylcyclopentan | 67 |
| 11) Ölsäuremethylester | 15 bar/30°C | 1-Decen<br>9-Decensäuremethylester | 44<br>46 |
| 12) Linolsäureäthylester | 15 bar/40°C | 1-Hepten<br>1,4-Pentadien<br>9-Decensäureäthylester | 33<br>31<br>21 |
| 13) Geranylaceton** | 14 bar/-5°C | Isobutylen<br>2-Methyl-1,5-hexadien<br>4-(1-Butenyl)methyl-keton | 33<br>28<br>29 |

* 2,4,4-Trimethyl-2-penten
** $(CH_3)_2C = CH(CH_2)_2C(CH_3) = CH(CH_2)_2C(=O)CH_3$
(a) Summe der jeweiligen Olefine entspricht dem Umsatz

# EP 0 522 067 B1

Tabelle 2

| Äthenolyse von Olefinen mit $(CH_3)_4 Re_2 O_4$ auf $Al_2 O_3/SiO_2$ | | | |
|---|---|---|---|
| Beispiel/Ausgangsmaterial | Reaktionsbedingungen | Produkte | Ausbeuten [a] (%) |
| 14) cis/trans-3-Hepten | 10 bar/40 °C | 1-Buten<br>1-Penten | 35<br>35 |
| 15) 1,3-Cyclohexadien | 10 bar/30 °C | 1,5-Hexadien<br>1,3-Butadien<br>1,3,7-Octatrien | 34<br>30<br>10 |
| 16) Cyclopenten | 20 bar/30 °C | 1,6-Heptadien | 75 |
| 17) Ölsäuremethylester | 15 bar/30 °C | 1-Decen<br>9-Decensäuremethylester | 46<br>44 |

[a] Summe der jeweiligen Olefine entspricht dem Umsatz

**Patentansprüche**

1. Verwendung von Verbindungen der allgemeinen Formel $R^1_a Re_b O_c$ (I), worin a 1 bis 6, b 1 bis 4 und c 1 bis 14 sind und die Summe von a, b und c so ist, daß sie der 5- bis 7-Wertigkeit des Rheniums gerecht wird mit der Maßgabe, daß c nicht größer als 3•b ist, und worin $R^1$ einen Alkylrest mit 1 bis 9 C-Atomen, einen Cycloalkylrest mit 5 bis 10 C-Atomen oder einen Aralkylrest mit 7 bis 9 C-Atomen darstellt, wobei $R^1$ wenigstens teilweise fluoriert sein kann, die Verbindungen nicht mehr als drei Gruppen mit mehr als 6 C-Atomen je Rheniumatom enthalten und an das C-Atom in $\alpha$-Stellung noch wenigstens ein Wasserstoffatom gebunden ist, die auf oxydische Trägermaterialien aufgebracht sind, als Katalysatoren zur äthenolytischen Metathese olefinischer Verbindungen.

2. Verfahren zur äthenolytischen Metathese von olefinischen Verbindungen, dadurch gekennzeichnet, daß man Olefine des Typs $YCZ = CZ\text{-}(CX_2)_n R^2$ (II), worin n eine ganze Zahl von 1 bis 28, X H oder F ist, Y H oder Alkyl mit 1 bis 10 C-Atomen ist und Z H oder einen nichtaromatischen Kohlenwasserstoffrest mit 1 bis 6 C-Atomen darstellt, Y und Z jedoch nicht gleichzeitig Wasserstoff sind, und der Substituent $R^2$ H, Alkyl, Halogen, $COOR^3$ oder $OR^4$ ist, worin $R^3$ und $R^4$ Alkyl mit 1 bis 15, vorzugsweise 1 bis 6, Kohlenstoffatomen oder Phenyl sind, das am Ring noch 1 bis 3 Substituenten enthalten kann oder worin $R^4$ Trialkylsilyl $R^5_3$ Si ist, worin $R^5$ Alkyl mit 1 bis 5, vorzugsweise 1 bis 3, C-Atomen darstellt, mit Äthylen an Katalysatoren umsetzt, die aus oxydischen Trägermaterialien bestehen, auf die Rheniumverbindungen der im Anspruch 1 definierten Art aufgebracht sind.

3. Verfahren nach Anspruch 2, dadurch gekennzeichnet, daß der Katalysatorträger im wesentlichen aus Aluminiumoxid, Siliziumdioxid, Titan-, Zirkon-, Niob-, Tantal-, Chromoxid oder Kombinationen davon besteht.

4. Verfahren nach Anspruch 2 oder 3, dadurch gekennzeichnet, daß man bei einem Äthylendruck von 3 bis 30, vorzugsweise von 5 bis 20 bar arbeitet.

5. Verfahren nach einem oder mehreren der Ansprüche 2 bis 4, dadurch gekennzeichnet, daß man bei einer Temperatur von -25 bis +70, vorzugsweise von +20 bis +65 °C arbeitet.

6. Verfahren nach einem oder mehreren der Ansprüche 2 bis 5, dadurch gekennzeichnet, daß das Olefin der Formel II ein Cycloolefin ist.

7. Verfahren nach einem oder mehreren der Ansprüche 2 bis 6, dadurch gekennzeichnet, daß das Olefin der Formel II ein funktionalisiertes Olefin ist.

8. Verfahren nach einem oder mehreren der Ansprüche 2 bis 7, dadurch gekennzeichnet, daß $R^4$ Alkyl mit 1 bis 6 Kohlenstoffatomen ist.

6

**9.** Verfahren nach einem oder mehreren der Ansprüche 2 bis 7, dadurch gekennzeichnet, daß $R^5$ Alkyl mit 1 bis 3 C-Atomen ist.

**10.** Ausführungsform nach einem oder mehreren der Ansprüche 1 bis 9, dadurch gekennzeichnet, daß der aktive Bestandteil in der Formel I Methylrheniumtrioxid $CH_3ReO_3$ ist.

## Claims

**1.** The use of compounds of the formula $R^1_aRe_bO_c$ (I), in which a is 1 to 6, b is 1 to 4 and c is 1 to 14 and the sum of a, b and c is such that it satisfies rhenium in its penta- to heptavalent states, with the proviso that c is not greater than 3•b, and in which $R^1$ is an alkyl radical with 1 to 9 carbon atoms, a cycloalkyl radical with 5 to 10 carbon atoms or an aralkyl radical with 7 to 9 carbon atoms, where $R^1$ may be at least partially fluorinated, the compounds do not contain more than three groups with more than 6 carbon atoms per rhenium atom and at least one hydrogen atom is bonded to the carbon atom in the $\alpha$-position, which are applied to oxidic support materials, as catalysts for the ethenolytic metathesis of olefinic compounds.

**2.** A process for the ethenolytic metathesis of olefinic compounds, which comprises reacting olefins of the type $YCZ = CZ\text{-}(CX_2)_nR^2$ (II), in which n is an integer from 1 to 28, X is H or F, Y is H or alkyl with 1 to 10 carbon atoms and Z is H or a non-aromatic hydrocarbon radical with 1 to 6 carbon atoms, Y and Z however not being hydrogen simultaneously, and the substituent $R^2$ is H, alkyl, halogen, $COOR^3$ or $OR^4$, in which $R^3$ and $R^4$ are alkyl with 1 to 15, preferably 1 to 6, carbon atoms or phenyl, which may also contain 1 to 3 substituents on the ring, or in which $R^4$ is trialkylsilyl $R^5_3Si$, in which $R^5$ is alkyl with 1 to 5, preferably 1 to 3, carbon atoms, with ethylene on catalysts which comprise oxidic support materials onto which rhenium compounds of the type defined in claim 1 are applied.

**3.** The process as claimed in claim 2, wherein the catalyst support essentially comprises aluminum oxide, silicon dioxide, titanium, zirconium, niobium, tantalum or chromium oxide or combinations of these.

**4.** The process as claimed in claim 2 or 3, wherein a pressure of 3 to 30, preferably of 5 to 20, bar of ethylene is used.

**5.** The process as claimed in one or more of claims 2 to 4, wherein a temperature of -25 to +70, preferably of +20 to +65°C is used.

**6.** The pocess as claimed in one or more of claims 2 to 5, wherein the olefin of formula II is a cycloolefin.

**7.** The process as claimed in one or more of claims 2 to 6, wherein the olefin of formula II is a functionalized olefin.

**8.** The process as claimed in one or more of claims 2 to 7, wherein $R^4$ is alkyl with 1 to 6 carbon atoms.

**9.** The process as claimed in one or more of claims 2 to 7, wherein $R^5$ is alkyl with 1 to 3 carbon atoms.

**10.** An embodiment as claimed in one or more of claims 1 to 9, wherein the active component in formula I is methylrhenium trioxide $CH_3ReO_3$.

## Revendications

**1.** Utilisation de composés de formule générale $R^1_aRe_bO_c$ (I), dans laquelle a a une valeur de 1 à 6, b de 1 à 4 et c de 1 à 14 et la somme de a, b et c satisfaisant à la valence 5 à 7 du rhénium et avec la condition que c ne soit pas supérieur à 3•b, et dans laquelle $R^1$ désigne un reste alkyle ayant de 1 à 9 atomes de carbone, un reste cycloalkyle ayant de 5 à 10 atomes de carbone ou un reste aralkyle ayant de 7 à 9 atomes de carbone, $R^1$ pouvant être au moins partiellement fluoré, composés qui n'ont pas plus de trois groupes ayant plus de 6 atomes de carbone par atome de rhénium et dans lesquels est encore lié au moins un atome d'hydrogène à l'atome de carbone de position $\alpha$, composés qui sont appliqués sur des oxydés servant de supports, comme catalyseurs de métathèse éthénolytique de composés oléfiniques.

**2.** Procédé de métathèse éthénolytique de composés oléfiniques, procédé caractérisé en ce que l'on fait réagir avec de l'éthylène des oléfines du genre $YCZ = CZ\text{-}(CX_2)_nR^2$ (II), dans laquelle n désigne un entier de 1 à 28, X l'hydrogène ou le fluor, Y l'hydrogène ou un alkyle ayant de 1 à 10 atomes de carbone et Z l'hydrogène ou un radical hydrocarboné non-aromatique ayant de 1 à 6 atomes de carbone, Y et Z ne pouvant toutefois être dans le même temps un hydrogène, et le substituant $R^2$ est l'hydrogène, un alkyle, un halogène ou un $COOR^3$ ou un $OR^4$, dans lesquels $R^3$ et $R^4$ sont des alkyles ayant de 1 à 15 atomes de carbone, de préférence de 1 à 6, ou un phényle dont le cycle peut encore porter de 1 à 3 substituants, ou dans lesquels $R^4$ est un trialkyl-silyle $R^5_3Si$, dans lequel $R^5$ est un alkyle ayant de 1 à 5 atomes de carbone, de préférence de 1 à 3, laquelle réaction se fait sur des catalyseurs formés d'oxydes comme supports, supports sur lesquels sont appliqués des composés du rhénium tels que définis à la revendication 1.

**3.** Procédé selon la revendication 2, caractérisé en ce que le support catalytique est essentiellement formé d'oxyde d'aluminium, de dioxyde de silicium, d'oxyde de titane, de zirconium, de niobium, de tantale, de chrome ou d'associations de plusieurs de ces oxydes.

**4.** Procédé selon la revendication 2 ou 3, caractérisé en ce que l'on opère sous une pression d'éthylène de 3 à 30 bar, de préférence de 5 à 20 bar.

**5.** Procédé selon une ou plusieurs des revendications 2 à 4, caractérisé en ce que l'on opère à une température de -25 à $+70°C$, de préférence de $+20$ à $+65°C$.

**6.** Procédé selon une ou plusieurs des revendications 2 à 5, caractérisé en ce que l'oléfine de formule II est une cyclooléfine.

**7.** Procédé selon une ou plusieurs des revendications 2 à 6, caractérisé en ce que l'oléfine de formule II est une oléfine fonctionnalisée.

**8.** Procédé selon une ou plusieurs des revendications 2 à 7, caractérisé en ce que $R^4$ désigne un alkyle ayant de 1 à 6 atomes de carbone.

**9.** Procédé selon une ou plusieurs des revendications 2 à 7, caractérisé en ce que $R^5$ désigne un alkyle ayant de 1 à 3 atomes de carbone.

**10.** Forme d'exécution selon une ou plusieurs des revendications 1 à 9, caractérisée en ce que le composé de formule I est le trioxyde de méthylrhénium $CH_3ReO_3$.